# FASCICULE DE BREVET EUROPEEN

(11) **EP 1 872 830 B1**
(45) Date de publication et mention de la délivrance du brevet: **05.08.2009**
(21) Numéro de dépôt: 07109064.1
(22) Date de dépôt: 29.05.2007
(51) Int. Cl.: A61Q 9/02, A61K 8/04, A61K 8/31, A61K 8/41, A61K 8/44

(54) **Gel de rasage automoussant sans savon à base de N-acylsarcosine; procédé de rasage**
Schaumbildendes Rasiergel ohne Seife auf Basis von N-Acylsarcosin; Rasierverfahren
Soap-free, self-foaming shaving gel based on N-acylsarcosine; shaving method

(30) Priorité: 23.06.2006 FR 0652622
(43) Date de publication de la demande: 02.01.2008
(73) Titulaire: L'ORÉAL, 75008 Paris (FR)
(72) Inventeur: Aubert, Lionnel, 95270, ASNIERES SUR OISE (FR); Dussault, Lydia, 78860, ST NOM LE BRETECHE (FR)
(74) Mandataire: Miszputen, Laurent

(56) Documents cités:
- WO-A-96/09032
- WO-A-02/087520
- WO-A-20/05094764
- GB-A- 2 325 936
- US-A- 5 902 574
- US-A1- 2003 053 980
- US-A1- 2005 112 084

## Description

La présente invention concerne un gel de rasage automoussant sans savon à base de N-acylsarcosine. Une telle composition est distribuée sous la forme d'un gel contenant un composant volatil qui produit un gel qui se transforme en mousse lors de l'étalement sur la peau pour la préparation d'un rasage humide au moyen d'un rasoir.

Sur le marché des produits de rasage en aérosol coexistent les mousses à raser et les gels de rasage automoussants.

Les gels de rasage automoussants sont biens connus et ont été décrits, par exemple, dans les brevets US2,995,521 ; US3,541,581, US4,405,489 ; US4,528,111; US4,651,503 ; US 5,248,495 ; US5,308,643 ; US5,326,556 et la demande de brevet WO91/07943. De telles formulations se présentent sous la forme d'une émulsion huile-dans-eau dans laquelle l'agent automoussant, généralement un hydrocarbure aliphatique volatil (ie : de faible point d'ébullition) est solubilisé dans la phase huileuse et la phase aqueuse comprend un savon hydrosoluble. Le produit est généralement conditionné dans un récipient aérosol avec une séparation telle qu'un piston ou une poche souple pour séparer l'agent automoussant de l'agent propulseur nécessaire pour l'expulsion du produit. Le produit est appliqué sous forme de gel transparent, translucide ou opaque qui est subtantiellement sans mousse jusqu'à l'étalement sur la peau, moment à partir duquel il se produit une mousse par évaporation de l'agent moussant hydrocarbure volatil.

Les gels de rasage automoussants conventionnels sont appréciés par une large gamme de consommateurs. Cependant, ces produits en raison de la présence de savon dans la composition ont tendance à assécher la peau et à augmenter la rugosité de celle-ci. Pour atténuer cet effet, les gels de rasage automoussants sont formulés avec des agents adoucissants comme des humectants, des émollients, des silicones, etc. L'incorporation de ces additifs influe sur les qualités esthétiques du produit et peut provoquer aussi par usage répété un desséchement de la peau. Pour ces raisons se sont développés des gels de rasage « sans savon » contenant des N-acylsarcosinates.

Les N-acylsarcosinates sont des tensioactifs anioniques bien connus de formule : 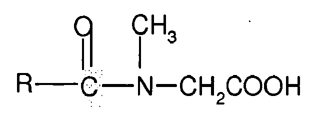 dans laquelle R est une chaîne hydrocarbonée d'acide gras. Ces tensioactifs sont généralement utilisés sous forme de sels hydrosolubles formés par neutralisation avec la soude, la potasse, l'ammoniaque ou la triéthanolamine et ont été préconisés dans une large gamme de produits comme les shampooings, les détergents, les dentifrices, les crèmes à raser ou les savons pour les mains. Par exemple, des crèmes à raser aérosol contenant des sarcosinates sont décrites dans les brevets US3,959,160 ; US4,113,643 et US4,140,648 ainsi que dans le document Harry's Cosmetology (7 ème édition, 1982 page 169 -voir Croda Cosmetic and Pharmaceutical Formulary Supplement, formula SV11). Une crème non aérosol de rasage pouvant contenir un N-acylsarcosinate a été décrite dans le brevet US4,892,729 et un gel de rasage non aérosol contenant un savon et un sarcosinate a été décrit dans le brevet US5,340,571.

Pour résoudre les inconvénients énoncés ci-dessus, on a proposé dans le brevet EP782436 des gels de rasage automoussants sans savon comprenant 65 à 85% d'eau, 4 à 16% en poids de N-acylsarcosine dans lequel le radical acyle est C₁₀-C₂₀, une base organique ou minérale dans une quantité suffisante pour solubiliser la N-acylsarcosine et produire un pH de 4 à 8, 1 à 8% en poids d'un agent automoussant et 1 à10% en poids d'une hydrocarbure liquide paraffinique non volatil.

On proposé dans la demande de brevet WO05/094764 des gels de rasage automoussants sans savon comprenant 65 à 85% d'eau, 4 à 16% en poids de N-acylsarcosine dans lequel le radical acyle est C₁₀-C₂₀, une base organique ou minérale dans une quantité suffisante pour solubiliser la N-acylsarcosine et produire un pH de 4 à 8, 1 à 8% en poids d'un agent automoussant et 0,25 à 5% en poids d'un polyol à chaîne courte en C₃-C₆ (glycérine, propylèneglycol).

Ces formules d'une part nécessitent la présence de N-acylsarcosine à des teneurs supérieures ou égales à 4% pour permettre la formation d'un gel de rigidité suffisante pour une bonne utilisation. D'autre part les qualités de la mousse obtenues lors de l'application sur la peau restent encore insuffisantes.

La demanderesse a découvert de manière surprenante que l'on pouvait obtenir des gels automoussants de rigidité acceptable et ayant de bonnes propriétés moussantes sans savon sans les inconvénients énoncés précédemment en utilisant une N-acylsarcosine à des teneurs inférieures à 4 % en présence d'une base minérale ou organique dans une quantité suffisante pour solubiliser la N-acylsarcosine et produire un pH de 4 à 8; au moins un tensioactif anionique, au moins un tensioactif non-ionique, au moins un agent automoussant.

La présente invention concerne donc un gel de rasage automoussant sans savon comprenant dans un milieu cosmétiquement acceptable
a) au moins une phase aqueuse
b) au moins une N-acylsarcosine où le radical acyle est en C₁₀-C₂₀ dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif anionique ;
e) au moins un tensioactif non-ionique;
f) au moins un agent automoussant.

La présente invention concerne un procédé de rasage consistant à appliquer sur la peau un gel de rasage automoussant tel que défini ci-dessus.

L'invention concerne également un kit de rasage caractérisé par le fait qu'il comprend
(a) au moins un gel de rasage automoussant tel que défini ci-dessus et
(b) au moins un rasoir notamment jetable et/ou
(c) un moyen d'étalement d'une composition de rasage.

On entend par « sans savon » comme contenant moins de 1% en poids de savon.

On entend par « milieu cosmétiquement acceptable » par on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Les N-acylsarcosines conformes à l'invention sont choisies de préférence parmi celles ayant un radical acyle en C₁₂-C₁₈. Plus préférentiellement, elles sont choisies parmi stéaroyl sarcosine, myristoyl sarcosine, oleoyl sarcosine, lauroyl sarcosine, cocoyl sarcosine ou leurs mélanges. Encore plus préférentiellement, elles sont choisies parmi stéaroyl sarcosine, myristoyl sarcosine et leurs mélanges. La ou les sarcosines sont présents à des teneurs inférieures à 4% en poids et de préférence allant de 2,5 à 3,5% par rapport au poids total de la composition.

Selon une forme particulière de l'invention, on peut utiliser un sarcosinate pré-neutralisé. Dans ce cas, il ne sera pas nécessaire d'ajouter séparément la base à la composition sauf s'il faut ajuster le pH de la composition comme souhaité.

La base peut être choisie parmi les bases minérales comme la potasse, la soude, l'ammoniaque. Elle peut être choisie parmi les bases organiques en particulier les alcanolamines telles que isopropanolamine, mono-, di- et triéthanolamine, aminoethylpropanol et aminomethylpropanol. La triéthanolamine est préférentielle. La quantité de base utilisée dépend de la quantité de sarcosine présente dans la composition. Une quantité suffisante de base doit être utilisée pour solubiliser la sarcosine dans la phase aqueuse et produire un pH de 4 à 8 et plus préférentiellement de 5 à 7. Pour atteindre cette plage de pH, la sarcosine est de préférence neutralisée de 50 à 90%, plus préférentiellement de 60 à 80%. On utilisera de préférence la sarcosine en léger excès molaire par rapport à la base. La base est de préférence présente à un taux variant de 1 à 6% par rapport au poids total de la composition.

La phase aqueuse des compositions selon l'invention représente de préférence de 65 à 85% en poids du poids total de la composition et plus préférentiellement de 70 à 80% en poids.

On peut utiliser par exemple comme tensioactifs anioniques conformes à l'invention, les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

Comme carboxylates, on peut citer par exemple :
- les amido éthercarboxylates (AEC), comme le lauryl amido ether carboxylate de sodium (3 OE) commercialisé sous la dénomination AKYPO FOAM 30® par la société Kao Chemicals ;
- les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxyméthyle, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX® par la société Nikkol ;
- les acétates tels que le 2-(2-hydroxy alkyloxy)acétate de sodium commercialisé sous la dénomination BEAULIGHT SHAA par la société Sanyo ;
- les alaninates comme le N-lauroyl-N-methylamidopropionate de sodium, commercialisé sous la dénomination SODIUM NIKKOL ALANINATE LN 30® par la société Nikkol ou sous la dénomination ALANONE ALE® par la société Kawaken, et le N-lauroyl N-methylalanine triéthanolamine, commercialisé sous la dénomination ALANONE ALTA® par la société Kawaken ; (3) les acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; (4) les aspartates comme le mélange de N-lauroylaspartate de triéthanolamine et de N-myristoylaspartate de triéthanolamine, commercialisé sous la dénomination ASPARACK® par la société Mitsubishi ; (5) les glycinates comme le N-cocoyl glycinate de sodium commercialisé sous les dénominations AMILITE GCS-12® et AMILITE GCK 12 par la société Ajinomoto ;
- les citrates tels que le mono-ester citrique d'alcools de coco oxyéthylénés (9 OA) commercialisé sous la dénomination WITCONOL EC 1129 par la société Goldschmidt ;
- les galacturonates comme le Dodecyl D- galactoside uronate de sodium commercialisé par la société SOLIANCE ;

Comme alkyl sulfates oxyéthylénés ou non, on peut citer par exemple le lauryl éther sulfate de sodium (C12-14 70/30) (2,2 OE) commercialisé sous la dénomination SIPON AOS 225® par la société Cognis, le lauryl éther sulfate d'ammonium (C12-14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le mélange de lauryl et oleyl éther sulfate de sodium et magnésium commercialisé sous la dénomination EMPICOL BSD 52 par la société Albright & Wilson.

Comme sulfonates, on peut citer par exemple (1) les alpha-oléfines sulfonates comme l'alpha-oléfine sulfonate de sodium (C14-16) commercialisé sous la dénomination BIO-TERGE AS-40 pa s dénominations BIO-TERGE AS-40® et dénomination BIO-TERGE AS-40® par la société Stepan, sous les dénominations WITCONATE AOS PROTEGE® et SULFRAMINE AOS PH 12® par la société Witco, l'oléfine sulfonate de sodium secondaire commercialisé sous la dénomination HOSTAPUR SAS 30® par la société Clariant ; (2) les iséthionates comme le cocoyl-iséthionate de sodium, tel que le produit commercialisé sous la dénomination JORDAPON CI P® par la société Jordan, (3) les taurates comme le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo.

Comme phosphates, on peut citer par exemple les monoalkylphosphates et les dialkylphosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea.

Comme polypeptides (qui sont des composés obtenus par condensation d'une chaîne grasse sur les aminoacides de céréale et notamment du blé et de l'avoine), on peut citer par exemple le sel de potassium de la lauroyl protéine de blé hydrolysée, commercialisé sous la dénomination AMINOFOAM W OR® par la société Croda ; le sel de triéthanolamine de cocoyl protéine de soja hydrolysée, commercialisé sous la dénomination MAY-TEIN SY® par la société Maybrook ; le sel de sodium des lauroyl amino-acides d'avoine, commercialisé sous la dénomination PROTEOL OAT® par la société Seppic ; l'hydrolysat de collagène greffé sur l'acide gras de coprah, commercialisé sous la dénomination GELIDERM 3000® par la société Deutsche Gelatine ; les protéines de soja acylées par des acides de coprah hydrogénés, commercialisées sous la dénomination PROTEOL VS 22® par la société Seppic.

Comme dérivés anioniques d'alkyl-polyglucosides, on peut citer notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia ; le sel di-sodique d'ester sulfosuccinique de cocoylpolyglucoside (1,4), commercialisé sous la dénomination ESSAI 512 MP® par la société Seppic ; le sel de sodium d'ester citrique de cocoyl polyglucoside (1,4) commercialisé sous la dénomination EUCAROL AGE-EC® par la société Cesalpinia, le Lauryl polyglucoside éther carboxylate de sodium commercialisé sous la dénomination PLANTAPON LGC SORB par la société Cognis.

On utilisera de préférence les sels d'alkyléthersulfates en C₆-C₂₄ comportant de 1 à 30 groupements oxyde d'éthylène , en particulier les sels de métaux alcalins ou alcalino-terreux, d'ammonium, d'amine ou d'aminoalcool et plus particulièrement les sels de sodium et encore plus particulièrement les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate (nom CTFA).

Les compositions selon l'invention contiennent un ou plusieurs tensioactifs non ioniques. Ce sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools gras dont la chaîne grasse comporte de préférence de 8 à 20 atomes de carbone ; les alcools, les alpha-diols, les alkylphénols ou les acides gras polyéthoxylés, polypropoxylés ou polyglycérolés, ayant une chaîne grasse comportant, de préférence, de 8 à 20 atomes de carbone, et où le nombre de groupements oxyde d'éthylène ou oxyde de propylène varie de préférence de 2 à 60 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant de préférence en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de préférence de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Cognis, et sous la dénomination ORAMIX NS 10® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

Les tensioactifs non-ioniques préférentiels sont choisis parmi :
- les alcools gras ont la chaîne grasse est en C₈-C₂₀, plus préférentiellement dont la chaîne grasse est en C₁₂-C₁₈ comme par exemple l'alcool myristique, l'alcool laurique, l'alcool stéarylique et l'octyldodécanol ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène. Parmi ces composés, on peut citer par exemple Oleth-20, Steareth-21, Ceteth-20, Laureth-4, Laureth-23. De façon plus préférentielle, on choisira un mélange de Laureth-4 et Laureth-23.

Le ou les tensioactifs non-ioniques sont présents de préférence à des concentrations allant de 5 à 20% en poids et plus préférentiellement de 7 à 12% en poids par rapport au poids total de la composition.

L'agent automoussant est choisi de préférence parmi les hydrocarbures volatils et les hydrocarbures volatils halogénés ayant un point d'ébullition suffisamment bas pour permettre à ces derniers de s'évaporer et de mousser le gel à l'application sur la peau et un point d'ébullition suffisamment haut pour éviter de produire une mousse prématurément. Le point d'ébullition de l'agent automoussant varie de préférence de -20 à 40 °C. L'agent automoussant est choisi de préférence de telle sorte à former une pression de vapeur à 20 °C de 3 à 20 psig et de préférence de 5 à 15 psig. Les agents automoussants utilisables selon l'invention sont choisis parmi les hydrocarbures aliphatiques en C₄-C₆ tels que n-pentane, isopentane, néopentane, n-butane, isobutane et leurs mélanges. Plus préférentiellement, on utilisera un mélange isopentane/isobutane dans un rapport en poids allant de 1/1 à 3/1. L'agent automoussant est de préférence présent à des concentrations allant de 1 à 8% en poids et plus préférentiellement de 2 à 5% en poids par rapport au poids total de la composition.

Les compositions de l'invention peuvent contenir en plus un agent gélifiant et par exemple contenir au moins un hydrocarbure liquide non volatil. Les termes « volatil » et « liquide » signifient que ces matériaux sont liquides à température ambiante et ont un point d'ébullition au dessus de 200 °C. Parmi ces hydrocarbures liquides, on peut citer les huiles minérales, les liquides aliphatiques ramifiés. Ces liquides ont de préférence de 16 à 48 atomes de carbone, plus préférentiellement de 20 à 40 atomes de carbone et une viscosité cinétique (mesurée selon la norme ASTM D445) de 5 à 100 cst et plus préférentiellement de 10 à 70 cst à 40 °C. Les hydrocarbures liquides non-volatils préférentiels sont choisis parmi les huiles minérales ayant une viscosité cinétique de 10 à 70 cst, les polyisobutènes hydrogénés de poids moléculaire de 320 à 420 et leurs mélanges. Le ou les hydrocarbures liquides non volatils sont de préférence présents à des concentrations inférieures ou égales à 10% et de préférence inférieures ou égales à 7% en poids par rapport au poids total de la composition.

Les compositions de l'invention peuvent également contenir un agent gélifiant auxiliaire hydrosoluble ou un agent épaississant pour améliorer la consistance et la stabilité du gel ou pour ajuster la viscosité.

Parmi ces agents gelifiants auxiliaires, on peut citer les polymères hydroxyalkylcelluloses comme hydroxyethylcellulose ou hydroxypropylcellulose (produits vendus respectivement sous la dénomination commerciale NATROSOL ou KLUCEL) ; les copolymères d'acide acrylique et de polyallylsucrose (produits vendus sous la dénomination commerciale CARBOPOL) ; la carboxymethylcellulose et ka cellulose methyl ether (produits vendus sous la dénomination commerciale METHOCEL) ; les gommes naturelles ou synthétiques, les amidons. Les agents épaississants préférentiels sont choisis parmi hydroxyethylcellulose ou hydroxypropylcellulose ou leurs mélanges. Les agents gelifiants auxiliaires ou épaississants sont de préférence présents à des concentrations allant de 0,01 à 5% en poids, plus préférentiellement de 0,05 à 2% en poids et encore plus préférentiellement de 0,01 à 2% en poids par rapport au poids total de la composition.

Les compositions de l'invention peuvent contenir en plus un polyol à chaîne courte pour améliorer les qualités de mousse et/ou la stabilité de la composition. Le ou les polyols sont de préférence présents à des concentrations inférieures à 5% en poids et plus préférentiellement allant de 0,25 à 5% en poids par rapport au poids total de la composition. Parmi les polyols utilisables ont peut citer la glycérine, le propylène glycol ou leurs mélanges.

Les compositions de rasage selon l'invention peuvent contenir en plus une variété d'ingrédients cosmétiques classiques pour améliorer les qualités esthétiques et les performances de ces compositions.

Les compositions selon l'invention peuvent également contenir en plus un polymère cationique conditionneur pour améliorer la lubricité et le toucher de la peau après rasage. On peut citer par exemple les sels d'ammonium quaternaires de l'hydroxyyethylcellulose comme Polyquaternium-10, Polyquaternium-24.

On peut également citer les polymères cationiques suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.
On peut aussi utiliser comme polymère cationique les guars cationiques telles que le produit JAGUAR commercialisé par la société RHODIA.

Le ou les polymères cationiques conditionneurs sont présents de préférence à des concentrations allant de 0,05 à 2% en poids, plus préférentiellement allant de 0,1 à 1% en poids par rapport au poids total de la composition.

D'autres additifs peuvent également utilisés dans les compositions de l'invention comme
- les humectants comme le sorbitol ;
- les émollients tels que les esters gras comme l'isopropyl myristate, le decyl oleate, le 2-ethyhexyl palmitate, le PEG-7 Glyceryl Cocoate et le Glyceryl Linoleate ; les éthers gras propoxylés comme PPG-10 Cetyl Ether et PPG-11 Stearyl Ether ; les di- ou triglycérides comme la lécithine, le mélange de triglycérides caprique/caprylique, le PEG-10 Soy Sterol, les huiles végétales.
- les agents rafraichissants et les agents apaisants comme le menthol, l'aloe, l'allantoine, la lanoline, le bisabolol, l'acide hyaluronique ;
- les lubrifiants comme les polyethyleneglycols (ie PEG-14M, PEG-23M), les tensioactifs fluorés, les silicones (ie : dimethicone, dimethiconol, dimethicone copolyol, stearyl dimethicone, cetyl dimethicone copolyol, cyclomethicone, etc...)
- les vitamines incluant les précurseurs et les dérivés comme le panthenol, le tocopheryl acetate, le niacinamide, le retinyl palmitate, le vitamine A palmitate ;
- les colorants ;
- les parfums ;
- les antioxydants ;
- les antibactériens et/ou les antifongiques ;
- les conservateurs (ie : methylchloroisothiazolinone, methylisothiazolinone, DMDM hydantoine, iodopropinyl butylcarbamate).

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions de rasage de la présente invention peuvent être conditionnées dans tout dispositif permettant de délivrer un gel automoussant. Par exemple, le dispositif peut être un récipient aérosol avec une séparation telle qu'un piston ou une poche souple pour séparer l'agent automoussant de l'agent propulseur nécessaire pour l'expulsion du produit. Le dispositif peut être également un tube souple ; un flacon pompe ou un flacon à paroi déformable.

L'invention concerne également un kit de rasage caractérisé par le fait qu'il comprend
(a) au moins un gel de rasage automoussant tel que défini ci-dessus et
(b) au moins un rasoir notamment jetable et/ou
(c) un moyen d'étalement d'une composition de rasage.

Selon une forme particulière de l'invention, le kit peut contenir en plus une composition après-rasage pour apaiser le feu du rasage.

Les exemples qui suivent servent à illustrer l'invention. Les quantités indiquées sont en % en poids sauf mention contraire, et les noms des composés en noms chimiques ou noms CTFA (International Cosmetic Ingredient Dictionary and Handbook) selon les cas.

### EXEMPLE

On prépare les gels de rasage sans savon automoussants suivants A et B (hors invention) qui sont dans des valves à poche.

| Ingrédients | Composition A | Composition B (hors invention) |
|---|---|---|
| BHT | 0,07 g | 0,07 g |
| Gluconate de Cuivre | 0,01 g | 0,01 g |
| Triéthanolamine | 0,05 g | 0,05 g |
| Bisabolol | 1,16 g | 1,16 g |
| 2,6-dimethyl-7-octen-2-ol | 0,39 g | 0,39 g |
| Paraffinum Liquidum | 0,96 g | 0,96 g |
| Polyquaternium-10 | 0,09 g | 0,09 g |
| Hydroxypropylcellulose | 0,09 g | 0,09 g |
| Isopentane et Isobutane (75/25) | 3,50 g | 3,50 g |
| Glycérine | 3,86 g | 3,86 g |
| Stearoyl sarcosine (and) Mytistoyl sarcosine (75%/25%) | 2,90 g | 2,90 g |
| Laureth-23 | 6,75 g | 6,75 g |
| Alcool myristylique | 2,41 g | 2,41 g |
| PEG-14M (Polyethylene Glycol 14.000 OE) | 0,19 g | 0,19 g |
| Laureth-4 | 0,48 g | 0,48 g |
| Sodium Laureth Sulfate à 2,2 OE en solution aqueuse à 70% | 7,72 g | - |
| Eau | qsp 100 g | qsp 100 g |
| Rigidité du Gel (en grammes) | 23 g | 2,5 g |

On mesure à 25°C la rigidité de chaque gel au moyen d'un analyseur de texture TA XT2i fabriqué par la société THERMO muni d'un cylindre SMS P/0-5 HS 0.5 inch diameter Hemi Spherical Delrin Cylinder Probe. On mesure la rigidité (exprimée en grammes) de chaque produit en compression par ledit cylindre à une vitesse de 2 mm/s sur une distance de 25 mm. On observe que la composition B de l'art antérieur ne contenant pas de tensioactif anionique et contenant moins de 4% en poids de sarcosine ne permet pas d'obtenir un gel rigide contrairement à la composition A de l'invention contenant un tensioactif anionique.

## Revendications

1. Gel de rasage automoussant sans savon comprenant dans un milieu cosmétiquement acceptable
a) au moins une phase aqueuse
b) au moins une N-acylsarcosine où le radical acyle est en C₁₀-C₂₀ dans une quantité inférieure à 4% en poids par rapport au poids total de la composition ;
c) au moins une base minérale ou organique dans une quantité suffisante pour solubiliser la N-acylsarcosine et produire un pH de 4 à 8 ;
d) au moins un tensioactif anionique ;
e) au moins un tensioactif non-ionique
f) au moins un agent automoussant.

2. Gel selon la revendication 1, où la N-acylsarcosine a un radical acyle en C₁₂-C₁₈.

3. Gel selon la revendication 1 ou 2, où la N-acylsarcosine est choisie parmi stéaroyl sarcosine, myristoyl sarcosine, oleoyl sarcosine, lauroyl sarcosine, cocoyl sarcosine ou leurs mélanges.

4. Gel selon la revendication 3, où la N-acylsarcosine est choisie parmi stéaroyl sarcosine, myristoyl sarcosine et leurs mélanges.

5. Gel selon l'une quelconque des revendications 1 à 4, où la ou les N-acylsarcosines sont présentes à des teneurs allant de 2,5 à 3,5% par rapport au poids total de la composition.

6. Gel selon l'une quelconque des revendications 1 à 5, où la base est choisie parmi les alcanolamines.

7. Gel selon la revendication 6, où la base est la triéthanolamine.

8. Gel selon l'une quelconque des revendications 1 à 7, où la sarcosine est neutralisée de 50 à 90%, plus préférentiellement de 60 à 80%.

9. Gel selon l'une quelconque des revendications 1 à 8, où la base est présente à un taux variant de 1 à 6% par rapport au poids total de la composition.

10. Gel selon l'une quelconque des revendications 1 à 9, où la phase aqueuse des compositions selon l'invention représente de préférence de 65 à 85% en poids du poids total de la composition et plus préférentiellement de 70 à 80% en poids.

11. Gel selon l'une quelconque des revendications 1 à 10, où le tensioactif anionique est choisi parmi les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

12. Gel selon la revendication 11, où le tensioactif anionique est choisi parmi les sels d'alkyléthersulfates en C₆-C₂₄ comportant de 1 à 30 groupements oxyde d'éthylène et plus particulièrement les sels d'alkyl (C₁₂-C₁₄)éthersulfates de sodium oxyéthylénés comportant un nombre moyen de groupes oxyde d'éthylène compris entre 1 et 4.

13. Gel selon l'une quelconque des revendications 1 à 12, où le tensioactif non-ionique est choisi parmi les alcools gras ; les alcools, les alpha-diols, les alkylphénols ou les acides gras polyéthoxylés, polypropoxylés ou polyglycérolés ; les copolymères d'oxyde d'éthylène et de propylène ; les condensats d'oxyde d'éthylène et de propylène sur des alcools gras ; les amides gras polyéthoxylés ; les amides gras polyglycérolés ; les amines grasses polyéthoxylées ; les esters d'acides gras du sorbitane éthoxylés; les esters d'acides gras du saccharose, les esters d'acides gras du polyéthylèneglycol ; les alkyl(C₆-C₂₄)polyglycosides ; les dérivés de N-alkyl(C₆-C₂₄)glucamine ; les oxydes d'amines et leurs mélanges.

14. Gel selon la revendication 13 où le ou les tensioactifs non-ioniques sont choisis parmi :
- les alcools gras dont la chaîne grasse est en C₈-C₂₀, plus préférentiellement dont la chaîne grasse est en C₁₂-C₁₈ ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène.
et leurs mélanges

15. Gel selon l'une quelconque des revendications 1 à 14, où le ou les tensioactifs non-ioniques sont présents à des concentrations allant de 5 à 20% en poids et plus préférentiellement de 7 à 12% en poids par rapport au poids total de la composition.

16. Gel selon l'une quelconque des revendications 1 à 14, où l'agent automoussant est choisi parmi les hydrocarbures volatils et les hydrocarbures volatils halogénés de point d'ébullition allant de -20 à 40 °C.

17. Gel selon la revendication 16, où l'agent automoussant est choisi parmi les hydrocarbures aliphatiques en C₄-C₆.

18. Gel selon la revendication 17, où l'agent automoussant est choisi parmi le n-pentane, isopentane, néopentane, n-butane, isobutane et leurs mélanges.

19. Gel selon la revendication 18, où l'agent automoussant est un mélange isopentane/isobutane dans un rapport en poids allant de 1/1 à 3/1.

20. Gel selon l'une quelconque des revendications 1 à 19, où l'agent automoussant est présent à des concentrations allant de 1 à 8% en poids et plus préférentiellement de 2 à 5% en poids par rapport au poids total de la composition.

21. Gel selon l'une quelconque des revendications 1 à 20, contenant en plus au moins un agent gélifiant.

22. Gel selon la revendication 21, où l'agent gélifiant est un hydrocarbure liquide non volatil.

23. Gel selon la revendication 22, où l'agent gélifiant est choisi parmi les huiles minérales, les liquides aliphatiques ramifiés ayant de 16 à 48 atomes de carbone, plus préférentiellement de 20 à 40 atomes de carbone et une viscosité cinétique de 5 à 100 cst et plus préférentiellement de 10 à 70 cst à 40 °C.

24. Gel selon la revendication 22, où le ou les hydrocarbures liquides non volatils sont présents à des concentrations inférieures ou égales à 10% et de préférence inférieures ou égales à 7% en poids par rapport au poids total de la composition.

25. Gel selon l'une quelconque des revendications 1 à 24, contenant en plus au moins un agent gélifiant auxiliaire hydrosoluble et/ou au moins un agent épaississant.

26. Gel selon la revendication 25, où l'agent gélifiant auxiliaire hydrosoluble ou l' agent épaississant sont choisis parmi les polymères hydroxyalkylcelluloses ; les copolymères d'acide acrylique et de polyallylsucrose ; la carboxymethylcellulose et la cellulose méthyl ether ; les gommes naturelles ou synthétiques, les amidons.

27. Gel selon la revendication 25 ou 26, où l'agent gélifiant auxiliaire hydrosoluble et/ou l'agent épaississant sont présents à des concentrations allant de 0,01 à 5% en poids, plus préférentiellement de 0,05 à 2% en poids et encore plus préférentiellement de 0,01 à 2% en poids par rapport au poids total de la composition.

28. Gel selon l'une quelconque des revendications 1 à 24, contenant en plus au moins un polyol à chaîne courte

29. Gel selon la revendication 28, où le ou les polyols sont présents à des concentrations inférieures à 5% en poids et plus préférentiellement allant de 0,25 à 5% en poids par rapport au poids total de la composition.

30. Gel selon la revendication 28 ou 29, où le ou les polyols sont choisis parmi la glycérine, le propylène glycol ou leurs mélanges.

31. Gel selon l'une quelconque des revendications 1 à 30, contenant en plus au moins un additif choisi parmi
- les polymères cationiques conditionneurs,
- les humectants,
- les émollients,
- les agents rafraîchissants et les agents apaisants,
- les lubrifiants,
- les vitamines incluant les précurseurs et les dérivés,
- les colorants ;
- les parfums ;
- les antioxydants ;
- les antibactériens et/ou les antifongiques;
- les conservateurs.

32. Procédé de rasage consistant à appliquer sur la surface de la peau à raser un gel de rasage automoussant tel que défini dans l'une quelconque des revendications précédentes puis à raser les poils au moyen d'un rasoir.

33. Kit de rasage **caractérisé par le fait qu'**il comprend
a) au moins un gel de rasage automoussant tel que défini dans l'une quelconque des revendications 1 à 31 ;
(b) au moins un rasoir notamment jetable et/ou
(c) un moyen d'étalement d'une composition de rasage.

34. Kit de rasage selon la revendication 33, contenant en plus une composition après-rasage pour apaiser le feu du rasage.

## Claims

1. Soap-free, self-foaming shaving gel comprising, in a cosmetically acceptable medium:
a) at least one aqueous phase;
b) at least one N-acylsarcosine where the acyl radical is a C₁₀-C₂₀ radical in an amount less than 4 wt% relative to the total weight of the composition;
c) at least one mineral or organic base in an amount sufficient to dissolve the N-acylsarcosine and produce a pH of 4 to 8;
d) at least one anionic surfactant;
e) at least one non-ionic surfactant; and
f) at least one self-foaming agent.

2. Gel according to Claim 1, where the N-acylsarcosine has a C₁₂-C₁₈ acyl radical.

3. Gel according to Claim 1 or 2, where the N-acylsarcosine is chosen from stearoyl sarcosine, myristoyl sarcosine, oleoyl sarcosine, lauroyl sarcosine, cocoyl sarcosine or mixtures thereof.

4. Gel according to Claim 3, where the N-acylsarcosine is chosen from stearoyl sarcosine, myristoyl sarcosine and mixtures thereof.

5. Gel according to any one of Claims 1 to 4, where the N-acylsarcosine or N-acylsarcosines are present at content levels ranging from 2.5 to 3.5% relative to the total weight of the composition.

6. Gel according to any one of Claims 1 to 5, where the base is chosen from alkanolamines.

7. Gel according to Claim 6, where the base is triethanolamine.

8. Gel according to any one of Claims 1 to 7, where the sarcosine is 50 to 90%, more preferentially 60 to 80%, neutralized.

9. Gel according to any one of Claims 1 to 8, where the base is present at a level varying from 1 to 6% relative to the total weight of the composition.

10. Gel according to any one of Claims 1 to 9, where the aqueous phase of the compositions according to the invention preferably represents from 65 to 85 wt%, and more preferentially from 70 to 80 wt%, of the total weight of the composition.

11. Gel according to any one of Claims 1 to 10, where the anionic surfactant is chosen from carboxylates, oxyethylenated or non-oxyethylenated alkyl sulphates, sulphonates, alkyl sulphoacetates, phosphates, polypeptides, anionic derivatives of alkyl polyglucoside, and mixtures thereof.

12. Gel according to Claim 11, where the anionic surfactant is chosen from the salts of C₆-C₂₄ alkyl ether sulphates having 1 to 30 ethylene oxide groups and more particularly the oxyethylenated sodium salts of C₁₂-C₁₄ alkyl ether sulphates having an average number of ethylene oxide groups between 1 and 4.

13. Gel according to any one of Claims 1 to 12, where the non-ionic surfactant is chosen from fatty alcohols; polyethoxylated, polypropoxylated or polyglycerolated alcohols, α-diols, alkylphenols or fatty acids; the copolymers of ethylene and propylene oxide; the condensates of ethylene and propylene oxide with fatty alcohols; polyethoxylated fatty amides; polyglycerolated fatty amides; polyethoxylated fatty amines; ethoxylated sorbitan fatty acid esters; sucrose fatty acid esters, polyethylene glycol fatty acid esters; (C₆-C₂₄) alkyl polyglycosides; N- (C₆-C₂₄)alkyl-glucamine derivatives; amine oxides and mixtures thereof.

14. Gel according to Claim 13, where the non-ionic surfactant or surfactants are chosen from:
- fatty alcohols having a C₈-C₂₀ fatty chain, more preferentially having a C₁₂-C₁₈ fatty chain;
- polyoxyethylenated ethers of fatty alcohols having a C₈-C₂₀ fatty chain, more preferentially having a C₁₂-C₁₈ fatty chain and having 2 to 60, more preferentially 2 to 30 ethylene oxide units,
and mixtures thereof.

15. Gel according to any one of Claims 1 to 14, where the non-ionic surfactant or surfactants are present at concentrations ranging from 5 to 20 wt% and more preferentially from 7 to 12 wt% relative to the total weight of the composition.

16. Gel according to any one of Claims 1 to 14, where the self-foaming agent is chosen from volatile hydrocarbons and halogenated volatile hydrocarbons having a boiling point ranging from -20 to 40°C.

17. Gel according to Claim 16, where the self-foaming agent is chosen from C₄-C₆ aliphatic hydrocarbons.

18. Gel according to Claim 17, where the self-foaming agent is chosen from n-pentane, isopentane, neopentane, n-butane, isobutane and mixtures thereof.

19. Gel according to Claim 18, where the self-foaming agent is an isopentane/isobutane mixture in a weight ratio ranging from 1/1 to 3/1.

20. Gel according to any one of Claims 1 to 19, where the self-foaming agent is present at concentrations ranging from 1 to 8 wt% and more preferentially from 2 to 5 wt% relative to the total weight of the composition.

21. Gel according to any one of Claims 1 to 20, comprising, in addition, at least one gelling agent.

22. Gel according to Claim 21, where the gelling agent is a non-volatile liquid hydrocarbon.

23. Gel according to Claim 22, where the gelling agent is chosen from mineral oils, branched aliphatic liquids having 16 to 48 carbon atoms, more preferentially 20 to 40 carbon atoms and a kinetic viscosity of 5 to 100 cst and more preferentially of 10 to 70 cst at 40°C.

24. Gel according to Claim 22, where the non-volatile liquid hydrocarbon or hydrocarbons are present at concentrations less than or equal to 10% and preferably less than or equal to 7% by weight relative to the total weight of the composition.

25. Gel according to any one of Claims 1 to 24, containing, in addition, at least one auxiliary water-soluble gelling agent and/or at least one thickening agent.

26. Gel according to Claim 25, where the auxiliary water-soluble gelling agent or the thickening agent is chosen from hydroxyalkyl cellulose polymers; acrylic acid/polyallyl sucrose copolymers; carboxymethyl cellulose and methyl ether cellulose; natural or synthetic gums; or starches.

27. Gel according to Claim 25 or 26, where the auxiliary water-soluble gelling agent and/or the thickening agent are present at concentrations ranging from 0.01 to 5 wt%, more preferentially from 0.05 to 2 wt% and even more preferentially from 0.01 to 2 wt% relative to the total weight of the composition.

28. Gel according to any one of Claims 1 to 24, containing, in addition, at least one short-chain polyol.

29. Gel according to Claim 28, where the polyol or polyols are present at concentrations less than 5 wt% and more preferentially ranging from 0.25 to 5 wt% relative to the total weight of the composition.

30. Gel according to Claim 28 or 29, where the polyol or polyols are chosen from glycerol, propylene glycol or mixtures thereof.

31. Gel according to any one of Claims 1 to 30, containing, in addition, at least one additive chosen from:
- cationic conditioning polymers;
- humectants;
- emollients;
- refreshing agents and soothing agents;
- lubricants;
- vitamin including precursors and derivatives;
- colorants;
- fragrances;
- antioxidants;
- antibacterial and/or antifungal agents; and
- preservatives.

32. Shaving method consisting in applying a self-foaming shaving gel as defined in any one of the preceding claims to the surface of the skin to be shaved, then in shaving the hairs using a razor.

33. Shaving kit **characterized in that** it comprises:
(a) at least one self-foaming shaving gel as defined in any one of Claims 1 to 31;
(b) at least one, especially disposable, razor; and/or
(c) la means of spreading a shaving composition.

34. Shaving kit according to Claim 33, containing, in addition, an after-shave composition to soothe razor burn.

## Patentansprüche

1. Selbstschäumendes seifenfreies Rasiergel, das in einem kosmetisch akzeptablen Medium enthält:
a) mindestens eine wässrige Phase,
b) mindestens ein N-Acylsarcosin, bei dem die Acylgruppe 10 bis 20 Kohlenstoffatome aufweist, in einer Menge unter 4 Gew,-%, bezogen auf das Gesamtgewicht der Zusammensetzung,
c) mindestens eine organische oder anorganische Base in einer Menge, die ausreichend ist, um das N-Acylsarcosin zu solubilisieren und einen pH-Wert von 4 bis 8 einzustellen,
d) mindestens einen anionischen grenzflächenaktiven Stoff,
e) mindestens einen nichtionischen grenzflächenaktiven Stoff,
f) mindestens einen selbstschäumenden Stoff,

2. Gel nach Anspruch 1, wobei das N-Acylsarcosin eine C₁₂-₁₈-Arylgruppe aufweist,

3. Gel nach Anspruch 1 oder- 2, wobei das N-Acylsarcosin unter Stearoylsarcosin, Myristoylsarcosin, Oleoylsarcosin, Lauroylsarcosin, Cocoylsarcosin oder deren Gemsichen ausgewählt ist.

4. Gel nach Anspruch 3, wobei das N-Acylsarcosin unter Stearoylsarcosin, Myristoylsarcosin und deren Gemsichen ausgewählt ist.

5. Gel nach einem der Ansprüche 1 bis 4, wobei das oder die N-Acylsarcosin(e) in Mengenanteilen von 2,5 bis 3,5 %, bezogen auf das Gesamtgewicht der Zusammensetzung, enthalten sind.

6. Gel nach einem der Ansprüche 1 bis 5, wobei die Base unter den Alkanolaminen ausgewählt ist.

7. Gel nach Anspruch 6, wobei die Base das Triethanolamin ist,

8. Gel nach einem der Ansprüche 1 bis 7, wobei das Sarcosin zu 50 bis 90 % und noch bevorzugter 60 bis 80 % neutralisiert ist.

9. Gel nach einem der Ansprüche 1 bis 8, wobei die Base in einer Menge von 1 bis 6 %, bezogen auf das Gesamtgewicht der Zusammensetzung, enthalten ist.

10. Gel nach einem der Ansprüche 1 bis 9, wobei die wässrige Phase der erfindungsgemäßen Zusammensetzungen vorzugsweise 65 bis 85 Gew.-% des Gesamtgewichts der Zusammensetzung und noch bevorzugter 70 bis 80 Gew.-% ausmacht.

11. Gel nach einem der Ansprüche 1 bis 10, wobei der anionische grenzflächenaktive Stoff unter den Carboxylaten, Alkylsulfaten, die gegebenenfalls ethoxyliert sind, Sulfonaten, Alkylsulfoacetaten, Phosphaten, Polypeptiden, anionischen Alkylpolyglucosidderivaten und deren Gemischen ausgewählt ist.

12. gel nach Anspruch 11, wobei der anionische grenzflächenaktive Stoff unter den Salzen von Alkyl(C₆₋₂₄)ethersulfaten, die 1 bis 30 Ethylenoxidgruppen enthalten, und insbesondere Natriumsalzen von ethoxylierten Alkyl(C₁₂₋₁₄)ethersulfaten ausgewählt ist, die eine mittlere Anzahl an Ethylenoxidgruppen im Bereich von 1 bis 4 enthalten.

13. Gel nach einem der Ansprüche 1 bis 12, wobei der nichtionische grenzflächenaktive Stoff unter den Fettalkoholen; Alkoholen, Alphadiolen, Alkylphenolen oder Fettsäuren, die polyethoxyliert, polypropoxyliert oder mehrfach mit Glycerin verethert sind; Copolymeren von Ethylenoxid und Propylenoxid; Kondensaten von Ethylenoxid und Propylenoxid mit Fettalkoholen; polyethoxylierten Fettamiden; mehrfach mit Glycerin veretherten Fettamiden; polyethoxylierten Fettaminen; ethoxylierten Sorbitanfettsäureestern; Saccharosefettsäureestern; Polyethylenglycolfettsäureestern; Alkyl(C₆₋₂₄)polyglycosiden; N-Alkyl(C₆₋₂₄)glucaminderivaten; Aminoxiden und deren Gemischen ausgewählt ist.

14. Gel nach Anspruch 13, wobei der oder die nichtionischen grenzflächenaktiven Stoffe ausgewählt sind unter:
- Fettalkoholen, deren Fettkette 8 bis 20 Kohlenstoffatome aufweist und deren Fettkette vorzugsweise 12 bis 18 Kohlenstoffatome aufweist,
- polycthoxylierten Fettalkoholethern, deren Fettkette 8 bis 20 Kohlenstoffatome aufweist und deren Fettkette vorzugsweise 12 bis 18 Kohlenstoffatome aufweist und die 2 bis 60 und noch bevorzugter 2 bis 30 Ethylenoxidgruppen enthalten,
- und deren Gemischen.

15. Gel nach einem der Ansprüche 1. bis 14, wobei der oder die nichtionischen grenzflächenaktiven Stoffe in Konzentrationen von 5 bis 20 Gew.-% und vorzugsweise 7 bis 12 Gew.-%, bezogen auf das Gesamtgewicht der Zusammensetzung, enthalten sind.

16. Gel nach einem der Ansprüche 1 bis 14, wobei der selbstschäumende Stoff unter den flüchtigen Kohlenwasserstoffen und den halogenierten flüchtigen Kohlenwasserstoffen mit einem Siedepunkt von -20 bis 40 °C ausgewählt ist,

17. Gel nach Anspruch 16, wobei der selbstschäumende Stoff unter den aliphatischen C₄₋₆-Kohlenwasserstoffen ausgewählt ist.

18. Gel nach Anspruch 17, wobei der selbstschäumende Stoff unter n-Pentan, Isopentan, Neopentan, n-Butan, Isobutan und deren Gemischen ausgewählt ist.

19. Gel nach Anspruch 18, wobei der selbstschäumende Stoff ein Isopentan/Isobutan-Gemisch in einem Gewichtsverhältnis von 1/1 bis 3/1 ist.

20. Gel nach einem der Ansprüche 1 bis 19, wobei der selbstschäumende Stoff in Konzentrationen von 1 bis 8 Gew.-% und vorzugsweise 2 bis 5 Gel.-%, bezogen auf das Gesamtgewicht der Zusammensetzung, enthalten ist.

21. Gel nach einem der Ansprüche 1 bis 20, das ferner mindestens einen Gelbildner enthält.

22. Gel nach Anspruch 21, wobei es sich bei dem Gelbildner um einen nichtflüchtigen flüssigen Kohlenwasserstoff handelt.

23. Gel nach Anspruch 22, wobei der Gelbildner unter den Mineralölen, verzweigten aliphatischen Flüssigkeiten mit 16 bis 48 und vorzugsweise 20 bis 40 Kohlenstoffatomen und einer kinetischen Viskosität von 5 bis 100 cSt und noch bevorzugter 10 bis 70 cSt bei 40 °C ausgewählt ist.

24. Gel nach Anspruch 22, wobei der oder die nichtflüchtigen flüssigen Kohlenwvasserstoffe in Konzentrationen von höchstens 10 Gew.-% und vorzugsweise kleiner oder gleich 7 Gew,-%, bezogen auf das Gesamtgewicht der Zusammensetzung, enthalten sind.

25. Gel nach einem der Ansprüche 1 bis 24, das ferner mindestens einen ergänzenden wasserlöslichen Gelbildner und/oder mindestens ein Verdickungsmittel enthält.

26. Gel nach Anspruch 25, wobei der ergänzende wasserlösliche Gelbildner oder das Verdickungsmittel unter den Hydroxyalkylcellulosepolyrheren; Copolymeren von Acrylsäure und Polyallylsaccharose; Carboxymethylcellulose und Cellulosemethylether; natürlichen oder synthetischen Gummis; und Stärkeverbindungen ausgewählt list.

27. Gel nach Anspruch 25 oder 26, wobei der ergänzende wasserlösliche Gelbildner und/oder das Verdickungsmittel in Konzentrationen von 0,01 bis 5 Gew.-%, vorzugsweise 0,05 bis 2 Gel.-% und noch bevorzugter 0,01 bis 2 Gew.-%, bezogen auf das Gesamtgewicht der Zusammensetzung, enthalten sind,

28. Gel nach einem der Ansprüche 1 bis 24, das ferner mindestens ein kurzkettiges Polyol enthält.

29. Gel nach Anspruch 28, wobei das oder die Polyole in Konzentrationen unter 5 Gew.-% und vorzugsweise im Bereich von 0,25 bis 5 Gew.-%, bezogen auf das Gesamtgewicht der Zusammensetzung, enthalten sind.

30. Gel nach Anspruch 28 oder 29, wobei das oder die Polyole unter Glycerin, Propylenglycol oder deren Gemischen ausgewählt sind.

31. Gel nach einem der Ansprüche 1 bis 30, wobei sie ferner mindestens einen Zusatzstoff enthält, der ausgewählt ist unter:
- kationischen konditionierenden Polymeren,
- Fruchthultemitteln,
- Emollientien,
- erfrischenden Stoffen und beruhigenden Stoffen,
- Gleitmitteln,
- Vitaminen, wobei ihre Vorläufer und Derivate eingeschlossen sind,
- Farbmitteln
- Parfums,
- Antioxidantien,
- antibakteriellen Wirkstoffen und/oder antimykotischen Wirkstoffen,
- Konservierungsmitteln.

32. Verfahren zum Rasieren, das darin besteht auf die Oberfläche der Haut, die rasiert werden soll, ein selbstschäumendes Rasiergel auszutragen, wie es in einem der vorhergehenden Ansprüche definiert ist, und anschließend die Körperhaare mit einem Rasierer abzurasieren.

33. Kit für die Rasur, **dadurch gekennzeichnet, dass** es umfasst:
a) mindestens ein selbstschäumendes Rasiergel, wie es in einem der Ansprüche 1 bis 31 definiert ist;
b) mindestens einen Rasierer und insbesondere einen Wegwerfrasierer; und/ oder
c) ein Mittel zum Verteilen einer Zusammensetzung für die Rasur.

34. Kit für die Rasur nach Anspruch 33, das ferner eine Zusammensetzung enthält, die nach der Rasur aufgetragen wird und die durch die Rasur gereizte Haut beruhigt.
